# EUROPÄISCHE PATENTANMELDUNG

(11) **EP 2 700 978 A2**
(43) Veröffentlichungstag der Anmeldung: **26.02.2014**
(21) Anmeldenummer: 13178499.3
(22) Anmeldetag: 30.07.2013
(51) Int. Cl.: G01T 1/169

(54) **Messvorrichtung zur Dosismessung in der Strahlentherapie und Verfahren zum Überprüfen einer Strahlentherapievorrichtung**

(30) Priorität: 21.08.2012 DE 102012214820
(71) Anmelder: KUKA Laboratories GmbH, 86165 Augsburg (DE)
(72) Erfinder: Berke, Ralph, 86497 Horgau (DE)
(74) Vertreter: Patentanwälte Funk & Böss GbR

(57) **Zusammenfassung**

Die Erfindung betrifft eine Messvorrichtung zur Dosismessung in der Strahlentherapie und Verfahren zum Überprüfen einer Strahlentherapievorrichtung. Die Messvorrichtung umfasst ein Wasserphantom (21), eine mechanische Vorrichtung (2, 54), die eingerichtet ist, das Wasserphantom (21) zu bewegen, und eine Steuervorrichtung (3, 55). Das Wasserphantom (21) weist eine Detektorvorrichtung (23) auf, die eingerichtet ist, eine ionisierende Strahlung zu detektieren, und die Steuervorrichtung (3, 55) ist eingerichtet, die mechanische Vorrichtung (2, 54) derart anzusteuern, sodass diese das Wasserphantom (2) entsprechend einer Bewegung eines Lebewesens bewegt, die das Lebewesen während einer Bestrahlung mit einer Strahlentherapievorrichtung ausführt.

## Beschreibung

Die Erfindung betrifft eine Messvorrichtung zur Dosismessung in der Strahlentherapie und Verfahren zum Überprüfen einer Strahlentherapievorrichtung.

Strahlentherapievorrichtungen sind z.B. aus der GB 2471750 A bekannt. Diese umfassen üblicherweise eine Patientenliege, eine Trägervorrichtung beispielsweise in Form einer rotierbaren Gantry und eine an der Trägervorrichtung befestigte therapeutische Strahlungsquelle. Im bestimmungsgemäßen Betrieb der Strahlentherapievorrichtung erzeugt die therapeutische Strahlungsquelle eine ionisierende Strahlung, wie z.B. zur Strahlentherapie verwendete Röntgen- oder Gammastrahlung, oder eine hochernergetische Elektronen, Protonen oder Ionenstrahlung.

Um z.B. die Strahlentherapievorrichtung zu überprüfen oder eine Bestrahlung eines Lebewesens mittels der Strahlentherapievorrichtung zu planen, können z.B. aus der US 7,420,160 B2 bekannte Wasserphantome verwendet werden.

Die Aufgabe der Erfindung ist es, eine verbesserte Messvorrichtung zur Dosismessung in der Strahlentherapie anzugeben.

Eine weitere Aufgabe der Erfindung ist es, verbesserte Verfahren zum Überprüfen einer Strahlentherapievorrichtung anzugeben.

Die Aufgabe der Erfindung wird gelöst durch eine Messvorrichtung zur Dosismessung in der Strahlentherapie, aufweisend
- ein Wasserphantom mit einer Detektorvorrichtung, die eingerichtet ist, eine ionisierende Strahlung zu detektieren,
- eine mechanische Vorrichtung, die eingerichtet ist, das Wasserphantom zu bewegen, und
- eine Steuervorrichtung, die eingerichtet ist, die mechanische Vorrichtung derart anzusteuern, sodass diese das Wasserphantom entsprechend einer Bewegung eines Lebewesens bewegt, die das Lebewesen während einer Bestrahlung mit einer Strahlentherapievorrichtung ausführt.

Die weitere Aufgabe der Erfindung wird gelöst durch ein Verfahren zum Überprüfen einer Strahlentherapievorrichtung, aufweisend folgende Verfahrensschritte:
- Abstellen eines Wasserphantoms auf einer Patientenliege einer Strahlentherapievorrichtung, welche einen Roboterarm, eine Steuervorrichtung und eine Strahlungsquelle aufweist, wobei der Roboterarm mehrere, hintereinander folgende Glieder aufweist, die mittels Gelenke verbunden und relativ zueinander bezüglich Achsen beweglich sind, am Roboterarm die Patientenliege befestigt ist, die Steuervorrichtung eingerichtet ist, den Roboterarm zu bewegen, und das Wasserphantom eine Detektorvorrichtung aufweist, die eingerichtet ist, eine ionisierende Strahlung zu detektieren, und
- Bestrahlen des auf der Patientenliege abgestellten Wasserphantoms mittels der Strahlungsquelle und, gesteuert durch die Steuervorrichtung, gleichzeitiges Bewegen des Roboterarms, sodass das Wasserphantom eine Bewegung entsprechend einer Bewegung eines Lebewesens durchführt.

Die weitere Aufgabe der Erfindung wird auch gelöst durch ein Verfahren zum Überprüfen einer Strahlentherapievorrichtung, aufweisend folgende Verfahrensschritte:
- Abstellen eines Wasserphantoms und einer mechanischen Vorrichtung auf einer Patientenliege einer Strahlentherapievorrichtung, wobei die mechanischen Vorrichtung eingerichtet ist, das Wasserphantom zu bewegen,
- Bestrahlen des auf der Patientenliege abgestellten Wasserphantoms mittels einer Strahlungsquelle der Strahlentherapievorrichtung und gleichzeitiges Bewegen der mechanischen Vorrichtung, sodass das Wasserphantom eine Bewegung entsprechend einer Bewegung eines Lebewesens durchführt.

Die erfindungsgemäße Messvorrichtung umfasst demnach das Wasserphantom und die mechanische Vorrichtung. Wasserphantome als solche sind dem Fachmann bekannt und sind vorgesehen, z.B. während des Überprüfens einer Strahlentherapievorrichtung die Dosis der von der Strahlentherapievorrichtung erzeugten Strahlung zu messen. Das Wasserphantom umfasst z.B. einen mit einer Flüssigkeit gefüllten Behälter, in dem die Detektorvorrichtung angeordnet ist.

Die mechanische Vorrichtung ist vorgesehen, das Wasserphantom während des Bestrahlens zu bewegen.

Die mechanische Vorrichtung kann z.B. als ein Roboterarm ausgebildet sein, welcher mehrere, hintereinander folgende Glieder aufweist, die mittels Gelenke verbunden und relativ zueinander bezüglich Achsen beweglich sind. Der Roboterarm kann insbesondere Teil der zu überprüfenden Strahlungstherapievorrichtung sein und auch vorgesehen sein, eine Patientenliege für ein zu bestrahlendes Lebewesen zu halten. Somit kann für das Bestrahlen des Wasserphantoms dieses auf die Patientenliege abgestellt werden.

Die mechanische Vorrichtung kann auch als der mechanische Bestandteil eines Hexapods ausgebildet sein. Hexapods können relativ klein ausgeführt sein, sodass es möglich ist, das Wasserphantom mit dem Hexapod für die Bestrahlung auf die Patientenliege der Strahlungstherapievorrichtung abzustellen.

Erfindungsgemäß wird außerdem die mechanische Vorrichtung, insbesondere der Roboterarm bzw. der Hexapod derart bewegt, dass das Wasserphantom ein Bewegung ausführt, die einer Bewegung entspricht, die das Lebewesen während ihrer Bestrahlung durchführt. Die Bewegung des Lebewesens wird z.B. durch Atmung des Lebewesens hervorgerufen. Dadurch wird es ermöglicht, die Bestrahlung des Lebewesens mittels des Wasserphantoms verbessert zu simulieren. Aufgrund des Ergebnisses dieser Simulation bzw. aufgrund eines Auswerten der Detektorvorrichtung des Wasserphantoms nach dem Bestrahlen kann für das Bestrahlen des Lebewesens verbessert die Patientenliege bewegt werden, um zumindest teilweise die Bewegung des Lebewesens während der Bestrahlung zu kompensieren. Somit kann es insbesondere vorgesehen sein, die Patientenliege insbesondere mittels des Roboterarms während einer Bestrahlung des Lebewesens aufgrund der ausgewerteten Detektorvorrichtung zu bewegen.

Die Detektorvorrichtung kann vorzugsweise einen Grundkörper und wenigstens ein im oder am Grundkörper angeordnetes oder anordbares Detektormittel aufweisen. Der Grundkörper kann vorzugsweise aus einem Material bestehen, der die von der Strahlungsquelle der Strahlentherapievorrichtung erzeugten Strahlung zumindest annähernd genauso absorbiert wie das zu bestrahlende Körperteil des Lebewesens. Mittels der Strahlentherapievorrichtung soll insbesondere ein Tumor des Lebewesens bestrahlt werden. Die Absorptionsfähigkeit von Strahlung des Materials des Grundkörpers kann dann insbesondere der des Tumors zumindest annähernd entsprechen. Ein geeignetes Material ist beispielsweise Kork.

Vorzugsweise kann der Grundkörper einen inneren Grundkörper und einen äußeren Grundkörper aufweisen, welcher den inneren Grundkörper umschließt. Die Größe des inneren Grundkörpers kann dabei z.B. der Größe des zu bestrahlenden Tumors zumindest annähernd entsprechen. Die Absorptionsfähigkeit von Strahlung des Materials des inneren Grundkörpers kann dann insbesondere der des Tumors zumindest annähernd entsprechen. Die Absorptionsfähigkeit von Strahlung des Materials des äußeren Grundkörpers kann dann insbesondere des den Tumor umgebenden Gewebes zumindest annähernd entsprechen.

Das wenigstens eine Detektormittel kann beispielsweise wenigstens einen Röntgenfilm aufweisen. In diesem Fall kann es vorzugsweise vorgesehen sein, dass der Grundkörper wenigstens einen Schlitz aufweist, in dem der Röntgenfilm eingeschoben werden kann. Vorzugsweise weist der Grundkörper mehrere Schlitze auf, in die jeweils ein Röntgenfilm einschiebbar ist. Dann ist es möglich, eine dreidimensionale Strahlendosisverteilung nach dem Bestrahlen des Wasserphantoms zu erhalten.

Das wenigstens eine Detektormittel kann zusätzlich oder alternativ wenigstens einen im und/oder am Grundkörper angeordneten Strahlungsdetektor aufweisen. Dieser erzeugt z.B. während des Bestrahlens elektrische Signale, welcher der Strahlendosis zugeordnet sind. Diese können dann z.B. automatisiert ausgewertet werden.

Beispielsweise zur röntgenfreien Erfassung der Tumorposition des Lebewesens könnten beispielsweise Atemmodelle oder implantierbare Sonden mit externer Detektion z.B. auf der Hautoberfläche eingesetzt werden. Insbesondere mittels der erfindungsgemäßen Verfahren, die sich gegebenenfalls auf ein robotergestütztes Mess-Phantom beziehen, können individuellen Verhältnisse des Lebewesens bzw. des Patienten simuliert werden. Dieses robotergeführte Phantom bildet vorzugsweise ein physikalisches Mensch-Modell ab, das die potenzielle Tumorverschiebung im Zielgebiet simuliert und eine 3-D Messung der Strahlendosisverteilung i m Modellinnenraum zulässt.

Gegebenfalls führt ein Roboter oder Hexapod o. ä. dabei das Mess-Modell (Wasserphantom). Das Phantom ist insbesondere derart aufgebaut, dass z.B. Röntgenfilme zwischen einen scheibenartigen Phantomaufbau gebracht werden können. Zudem können gegebenenfalls im Modell verschiedene Gewebestrukturen simuliert werden, durch z.B. Einbringen von Kork oder anderen Materialien. Hierdurch kann ein möglichst realistisches physiologisches Modell für eine 3-D Dosimetrie des simulierten Tumors sowie der Tumorumgebung nachgebildet werden.

Bei Bedarf können die Daten aus der vorab simulierten Phantommessung mit den erfassten (online) Patienten-Sensormessdaten kombiniert werden, um daraus die potenzielle Tumorbewegung zu errechnen. Während des Bestrahlens kann so mittels eines entsprechenden Regelalgorithmus (mathematische Modelle) die Lage des Tumors vorausberechnet werden, um beispielsweise Rechenzeit einzusparen. Ebenso kann ein geeigneter Kompensationsvektor abgeleitet und über die Roboter-Gegenbewegung die aktuelle Tumorauslenkung kompensiert werden. Somit kann erreicht werden, dass eine konstante Tumorlage erreicht und damit eine konstante Dosisverteilung während der Bestrahlung des Lebewesens bewirkt werden.

Ausführungsbeispiele der Erfindung sind exemplarisch in den beigefügten schematischen Zeichnungen dargestellt. Es zeigen:
- Fig. 1: eine Strahlentherapievorrichtung,
- Fig. 2: ein Wasserphantom, das einen mit einer Flüssigkeit gefüllten Behälter und eine innerhalb des Behälters angeordnete Detektorvorrichtung aufweist,
- Figuren 3, 4: die Detektorvorrichtung in geschnittenen Darstellungen, und
- Fig. 5: eine weitere Strahlentherapievorrichtung.

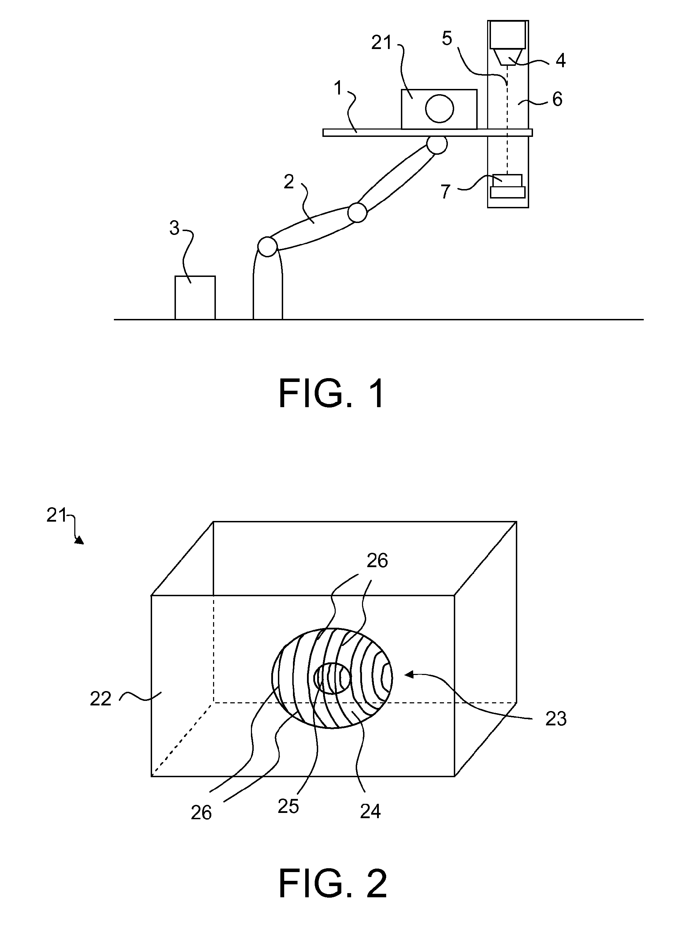

Die Figur 1 zeigt eine Strahlentherapievorrichtung. Diese umfasst im Falle des vorliegenden Ausführungsbeispiels eine Patientenliege 1 und einen Roboterarm 2, der mehrere, hintereinander folgende Glieder aufweist, die mittels Gelenke verbunden und relativ zueinander bezüglich Achsen beweglich sind. An einem Ende des Roboterarms 2 ist die Patientenliege 1 befestigt. Im Falle des vorliegenden Ausführungsbeispiels ist die Patientenliege 1 vorgesehen, dass auf dieser ein nicht näher gezeigtes Lebewesen liegen kann.

Der Roboterarm 2 weist Antriebe, insbesondere elektrische Antriebe auf, welche mit einer Steuervorrichtung 3 der Strahlentherapievorrichtung verbunden sind. Die elektrischen Antriebe sind vorzugsweise geregelte elektrische Antriebe. Mittels der Antriebe kann der Roboterarm 2 bzw. dessen Glieder relativ zueinander und gesteuert durch die Steuervorrichtung 3 bzw. durch ein auf der Steuervorrichtung 3 laufendes Rechnerprogramm bewegt werden.

Die Strahlentherapievorrichtung weist ferner eine therapeutische Strahlungsquelle 4 auf, welche beispielsweise mittels der Steuervorrichtung 3 gesteuert wird. Im bestimmungsgemäßen Betrieb der Strahlentherapievorrichtung erzeugt die therapeutische Strahlungsquelle 4 eine ionisierende Strahlung, wie z.B. zur Strahlentherapie verwendete Röntgen- oder Gammastrahlung, oder eine hochernergetische Elektronen, Protonen oder Ionenstrahlung, deren Zentralstrahl 5 in der Figur 1 gestrichelt gezeichnet ist. Die Strahlentherapievorrichtung umfasst ferner eine Trägervorrichtung 6, an der die therapeutische Strahlungsquelle 4 befestigt ist. Die Trägervorrichtung 6 ist z.B. als eine rotierbare Gantry ausgebildet, wie dies dem Fachmann im Prinzip bekannt ist.

Die Strahlentherapievorrichtung kann einen Strahlenempfänger 7 aufweisen, der ebenfalls an der Trägervorrichtung 6 befestigt ist und auf den die ionisierende Strahlung auftrifft. Mittels des Strahlenempfängers 7 können z.B. Portalbilder angefertigt werden.

Die Strahlentherapievorrichtung ist vorgesehen, einen Tumor des Lebewesens zu bestrahlen. Auf der Steuervorrichtung 3 läuft beispielsweise ein Rechenprogramm, mittels dem die Steuervorrichtung 3 den Roboterarm 2 derart ansteuert, sodass dieser beispielsweise während der Bestrahlung eine Bewegung des Lebewesens zumindest teilweise kompensiert.

Um im Falle des vorliegenden Ausführungsbeispiels beispielsweise eine Dosismessung für die Strahlentherapie durchzuführen und/oder vor der Bestrahlung des Lebewesens Daten zu erhalten, welche eine verbesserte Vorhersage der Bewegung des Lebewesens während der Bestrahlung erlauben, um beispielsweise die Patientenliege 1 mittels des Roboterarms 2 verbessert für das zumindest teilweise Kompensieren der Bewegung des Lebewesens zu bewegen, wird ein in der Fig. 2 in einer perspektivischen Darstellung dargestelltes Wasserphantom 21 verwendet.

Das Wasserphantom 21 ist vorgesehen, ionisierende Strahlung, wie z.B. die von der therapeutischen Strahlungsquelle 4 erzeugte ionisierende Strahlung hinsichtlich ihrer Strahlungsparameter und ihrer Wirkung auf den menschlichen Körper zu vermessen.

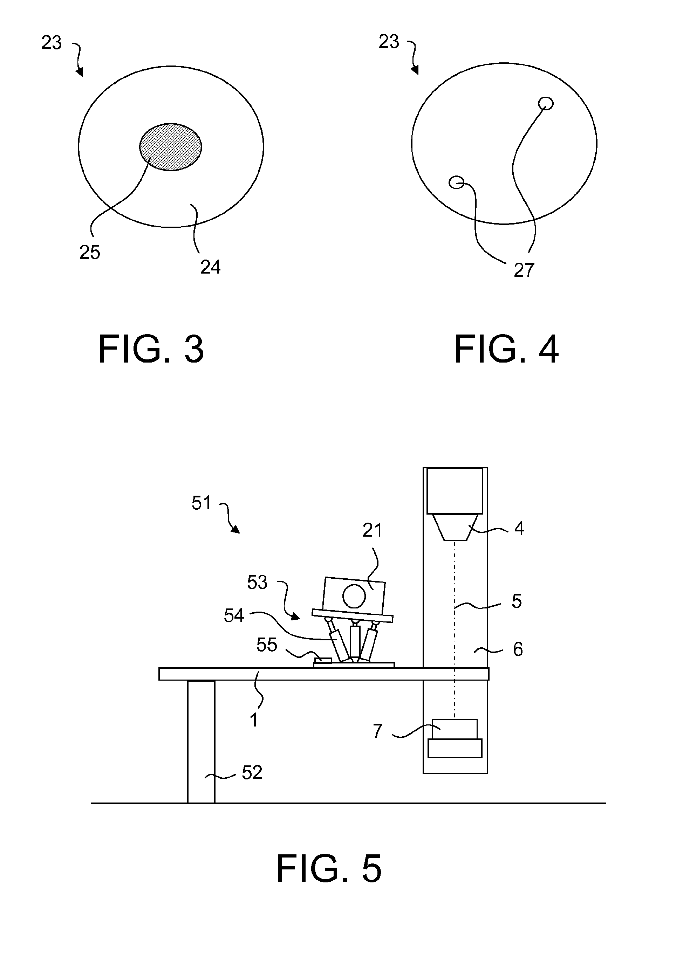

Im Falle des vorliegenden Ausführungsbeispiels weist das Wasserphantom 21 einen z.B. quaderförmig ausgeführten Behälter 22 auf, der insbesondere nach oben offen und mit einer Flüssigkeit, insbesondere mit Wasser, gefüllt ist. Das Wasserphantom 21 umfasst ferner eine Detektorvorrichtung 23, welche eingerichtet ist, die in die Flüssigkeit eindringende ionisierende Strahlung zu vermessen. Die Detektorvorrichtung 23 ist z.B. mit einer nicht näher dargestellten Haltevorrichtung am Behälter 22 befestigt. Die Figuren 3 und 4 zeigen die Detektorvorrichtung 23 in geschnittenen Darstellungen.

Im Falle des vorliegenden Ausführungsbeispiels umfasst die Detektorvorrichtung 23 einen insbesondere kugelförmig ausgebildeten äußeren Grundkörper 24, der einen inneren Grundkörper 25 umschließt. Der äußere Grundkörper 24 ist insbesondere aus einem Material gefertigt, welches einem mit der Strahlentherapievorrichtung zu bestrahlenden Körperteil des Lebewesens zugeordnet ist. Dieses Material absorbiert insbesondere die ionisierende Strahlung entsprechend dem Gewebe des Körperteils. Der äußere Grundkörper 24 ist beispielsweise aus Kork gefertigt. Der innere Grundkörper 24 ist insbesondere aus einem Material gefertigt, welches einem Tumor dieses Körperteils entspricht.

Die Detektorvorrichtung 23 weist im Falle des vorliegenden Ausführungsbeispiels wenigsten ein Detektormittel auf, mittels dem die ionisierende Strahlung gemessen werden kann. Bei dem Detektormittel handelt es sich beispielsweise um wenigstens einen Röntgenfilm 26, welcher in einen Schlitz bzw. die in Schlitze der beiden Grundkörper 24, 25 eingeführt werden kann bzw. eingeführt werden können. Dazu sind die beiden Grundkörper 24, 25 z.B. scheibenartig aufgebaut.

Zusätzlich oder alternativ kann als Detektormittel auch wenigstens ein Stahlungsdetektor 27 vorgesehen sein, welcher in den äußeren Grundkörper 24 und/oder inneren Grundkörper 25 angeordnet ist.

Im Falle des vorliegenden Ausführungsbeispiels wird das Wasserphantom 21 in Kombination mit dem Roboterarm 2 als eine Messvorrichtung zur Dosismessung in der Strahlentherapie verwendet. Das Wasserphantom 21 wird dazu auf die Patientenliege 1 gestellt und der Roboterarm 2 wird dazu mittels der Steuervorrichtung 3 automatisch derart bewegt, dass eine Bewegung des Lebewesens während der Bestrahlung simuliert wird. Die Bewegung des Lebewesens während der Bestrahlung erfolgt z.B. durch Atmung des Lebewesens. Um den Roboterarm 2 geeignet zu bewegen, läuft insbesondere auf der Steuervorrichtung 3 ein geeignetes Rechenprogramm, sodass die Steuervorrichtung 3 die Antriebe des Roboterarms 2 derart ansteuert, damit die Patientenliege 1 und somit das Wasserphantom 21 die simulierte Bewegung durchführt. Um die Bewegung des Lebewesens zu simulieren, ist z.B. in der Steuervorrichtung 3 ein mathematisches Bewegungsmodell des Lebewesens hinterlegt.

Während der Roboterarm 2 derart angesteuert wird, sodass das Wasserphantom 21 die simulierte Bewegung durchführt, wird das Wasserphantom 21 mittels der therapeutischen Strahlungsquelle 4 bestrahlt. Aufgrund dieser Bestrahlung wird das Wasserphantom 21 und insbesondere die Detektorvorrichtung 23 einer Strahlendosis ausgesetzt, welche mittels der Detektormittel, beispielsweise dem wenigstens einen Röntgenfilm 26 und/oder dem wenigstens einen Strahlungsdetektors 27 nach der Bestrahlung ausgewertet werden kann.

Somit ist es möglich, mittels des Roboterarms 2, welcher allgemein eine mechanische Vorrichtung zum Bewegen des Wasserphantoms 21 darstellt, vorzugsweise individuelle Patientenverhältnisse möglichst realitätsnah zu simulieren. Der Roboterarm 2, gesteuert durch die Steuervorrichtung 3, führt dabei das Wasserphantom 21 während der Phantom-Bestrahlung und simuliert die erwartete Bewegung des Tumors bzw. die Tumorverschiebung des Lebewesens. Die Bewegung des Tumors wird über das Wasserphantom 21 dargestellt. Die insbesondere runde Detektorvorrichtung 23 des Wasserphantoms 21 ist insbesondere so aufgebaut, dass z.B. Röntgenfilme 26 zwischen den beispielsweise scheibenartigen Kugelaufbau der Detektorvorrichtung 23 gebracht werden können. Zudem können im Kugelmodell, d.h. in der Detektorvorrichtung 23 verschiedene Gewebestrukturen simuliert werden, beispielsweise durch Einbringen von Kork und anderen Materialien. Hierdurch kann ein möglichst realistisches Modell für eine 3 D Dosimetrie des simulierten Tumors abgebildet werden.

Somit können z.B. die Daten aus einer alternativen Detektion der Tumorbewegung mit dem Ergebnis der Daten, welche mittels der simulierten Messung bei der 3D Dosimetrie erhalten wurden, kombiniert werden, um daraus z.B. eine perspektivische Tumorbewegung zu errechnen. Dadurch ist es z.B. möglich, die errechnete erwartete Tumorbewegung einem Regelalgorithmus zuzuführen, sodass während der Bestrahlung des Lebewesens mittels der Strahlentherapievorrichtung eine verbesserte, wenn nicht gar optimierte Bewegungskompensation der Tumorbewegung erreicht wird. Die Steuervorrichtung 3 könnte dann online, und bereits im Vorfeld, einen "Verschiebe-Vektor" der Tumorposition kalkulieren und mit den online Daten aus der "Alternativ-Detektion" abgleichen. Dergestalt könnte dann über eine gegenläufige Bewegung des Roboterarms 2 die Tumorbewegung zumindest teilweise kompensiert werden.

Die Figur 5 zeigt eine weitere Strahlentherapievorrichtung. Wenn nicht anders beschrieben, dass sind Bestandteile der in der Figur 5 gezeigten Strahlentherapievorrichtung, welche mit Bestandteile der in der Figur 1 gezeigten Strahlentherapievorrichtung im Wesentlichen bau- und funktionsgleich sind, mit denselben Bezugszeichen versehen.

Die in der Figur 5 gezeigte Strahlentherapievorrichtung unterscheidet sich im Wesentlichen von der in der Figur 1 dargestellte Strahlentherapievorrichtung dadurch, dass die Patientenliege 1 nicht mittels des Roboterarms 2 gehalten ist, sondern mittels einer Hubvorrichtung 52.

Um die in der Figur 5 dargestellte Strahlentherapievorrichtung zu überprüfen, kann eine weitere Messvorrichtung 51 zur Dosismessung in der Strahlentherapie verwendet werden. Diese umfasst im Falle des vorliegenden Ausführungsbeispiels das Wasserphantom 21 und als automatische mechanische Vorrichtung zum Bewegen des Wasserphantoms 21 einen Hexapod 53.

Neben dem mechanischen Bestandteil 54 des Hexapods 53, mittels dem das Wasserphantom 21 bewegt werden kann, umfasst der Hexapod 53 eine Steuervorrichtung 55, welche eingerichtet ist, den mechanischen Bestandteil 54 des Hexapods 53 derart zu bewegen, dass eine Bewegung des Lebewesens während der Bestrahlung simuliert wird. Die Bewegung des Lebewesens während der Bestrahlung erfolgt z.B. durch Atmung des Lebewesens. Um den mechanischen Bestandteil 54 des Hexapods 53 geeignet zu bewegen, läuft insbesondere auf der Steuervorrichtung 55 ein geeignetes Rechenprogramm, sodass die Steuervorrichtung 55 Antriebe des mechanischen Bestandteils 54 des Hexapods 53 derart ansteuert, damit das Wasserphantom 21 die simulierte Bewegung durchführt. Um die Bewegung des Lebewesens zu simulieren, ist z.B. in der Steuervorrichtung 55 ein mathematisches Bewegungsmodell des Lebewesens hinterlegt.

Während der Simulation steht der Hexapod 53 mit dem Wasserphantom 21 auf der Patientenliege 1 und wird mittels der therapeutischen Strahlungsquelle 4 bestrahlt.

## Patentansprüche

1. Messvorrichtung zur Dosismessung in der Strahlentherapie, aufweisend
- ein Wasserphantom (21) mit einer Detektorvorrichtung (23), die eingerichtet ist, eine ionisierende Strahlung zu detektieren,
- eine mechanische Vorrichtung (2, 54), die eingerichtet ist, das Wasserphantom (21) zu bewegen, und
- eine Steuervorrichtung (3, 55), die eingerichtet ist, die mechanische Vorrichtung (2, 54) derart anzusteuern, sodass diese das Wasserphantom (2) entsprechend einer Bewegung eines Lebewesens bewegt, die das Lebewesen während einer Bestrahlung mit einer Strahlentherapievorrichtung ausführt.

2. Messvorrichtung nach Anspruch 1, deren mechanische Vorrichtung als ein Roboterarm (2) ausgebildet ist, welcher mehrere, hintereinander folgende Glieder aufweist, die mittels Gelenke verbunden und relativ zueinander bezüglich Achsen beweglich sind.

3. Messvorrichtung nach Anspruch 1, bei der die mechanische Vorrichtung als der mechanische Bestandteil (54) eines Hexapods (53) ausgebildet ist.

4. Messvorrichtung nach einem der Ansprüche 1 bis 3, deren Wasserphantom (21) einen mit einer Flüssigkeit befüllbaren Behälter (22) aufweist, in dem die Detektorvorrichtung (23) angeordnet ist.

5. Messvorrichtung nach einem der Ansprüche 1 bis 4, bei der die Detektorvorrichtung (23) einen Grundkörper (24, 25) und wenigstens ein im und/oder am Grundkörper (24, 25) angeordnetes oder anordbares Detektormittel (26, 27) aufweist.

6. Messvorrichtung nach Anspruch 5, bei der der Grundkörper einen inneren Grundkörper (25) und einen äußeren Grundkörper (24) aufweist, welcher den inneren Grundkörper (25) umschließt.

7. Messvorrichtung nach Anspruch 5 oder 6, bei der das wenigstens eine Detektormittel wenigstens einen Röntgenfilm (26) aufweist, und insbesondere der Grundkörper (24, 25) wenigstens einen Schlitz aufweist, in dem der Röntgenfilm (26) bzw. in denen jeweils ein Röntgenfilm (26) einschiebbar ist.

8. Messvorrichtung nach einem der Ansprüche 5 bis 7, bei der das wenigstens eine Detektormittel wenigstens einen im und/oder am Grundkörper (24, 25) angeordneten Strahlungsdetektor (27) aufweist ist.

9. Verfahren zum Überprüfen einer Strahlentherapievorrichtung, aufweisend folgende Verfahrensschritte:
- Abstellen eines Wasserphantoms (21) auf einer Patientenliege (1) einer Strahlentherapievorrichtung, welche einen Roboterarm (2), eine Steuervorrichtung (3) und eine Strahlungsquelle (4) aufweist, wobei der Roboterarm (2) mehrere, hintereinander folgende Glieder aufweist, die mittels Gelenke verbunden und relativ zueinander bezüglich Achsen beweglich sind, am Roboterarm (2) die Patientenliege (1) befestigt ist, die Steuervorrichtung (2) eingerichtet ist, den Roboterarm (2) zu bewegen, und das Wasserphantom (21) eine Detektorvorrichtung (23) aufweist, die eingerichtet ist, eine ionisierende Strahlung zu detektieren, und
- Bestrahlen des auf der Patientenliege (1) abgestellten Wasserphantoms (21) mittels der Strahlungsquelle (4) und, gesteuert durch die Steuervorrichtung (3) gleichzeitiges Bewegen des Roboterarms (2), sodass das Wasserphantom (21) eine Bewegung entsprechend einer Bewegung eines Lebewesens durchführt.

10. Verfahren zum Überprüfen einer Strahlentherapievorrichtung, aufweisend folgende Verfahrensschritte:
- Abstellen eines Wasserphantoms (21) und einer mechanischen Vorrichtung (54) auf einer Patientenliege (1) einer Strahlentherapievorrichtung, wobei die mechanischen Vorrichtung (54) eingerichtet ist, das Wasserphantom (21) zu bewegen und insbesondere als ein Hexapod ausgeführt ist,
- Bestrahlen des auf der Patientenliege (1) abgestellten Wasserphantoms (21) mittels einer Strahlungsquelle (4) der Strahlentherapievorrichtung und gleichzeitiges Bewegen der mechanischen Vorrichtung (54), sodass das Wasserphantom (21) eine Bewegung entsprechend einer Bewegung eines Lebewesens durchführt.

11. Verfahren nach Anspruch 9 oder 10, zusätzlich aufweisend folgende Verfahrensschritte:
- Auswerten einer Detektorvorrichtung (23) des Wasserphantoms (23) nach dem Bestrahlen und
- Bewegen der Patientenliege (1) insbesondere mittels des Roboterarms (2) während einer Bestrahlung des Lebewesens aufgrund der ausgewerteten Detektorvorrichtung (23).
